(19) Europäisches Patentamt / European Patent Office / Office européen des brevets

(11) **EP 2 116 224 B1**

(12) **EUROPEAN PATENT SPECIFICATION**

(45) Date of publication and mention of the grant of the patent:
**27.01.2016 Bulletin 2016/04**

(21) Application number: **07849978.7**

(22) Date of filing: **27.11.2007**

(51) Int Cl.:
*A61K 8/73* (2006.01)     *A61K 8/02* (2006.01)
*A61K 8/34* (2006.01)     *A61Q 1/00* (2006.01)
*A61Q 5/00* (2006.01)     *A61Q 19/00* (2006.01)
*A61Q 19/10* (2006.01)

(86) International application number:
**PCT/JP2007/073275**

(87) International publication number:
**WO 2008/066193 (05.06.2008 Gazette 2008/23)**

(54) **COSMETIC COMPOSITION**

KOSMETISCHE ZUSAMMENSETZUNG

COMPOSITION COSMÉTIQUE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **27.11.2006 JP 2006317992**

(43) Date of publication of application:
**11.11.2009 Bulletin 2009/46**

(73) Proprietor: **JNC Corporation**
**Chiyoda-ku**
**Tokyo (JP)**

(72) Inventors:
- **YOSHIDA, Naoyuki**
  **Ichihara-shi**
  **Chiba 290-8551 (JP)**
- **YAMAMOTO, Yuichi**
  **Ichihara-shi**
  **Chiba 290-8551 (JP)**
- **ISHIDA, Kazushi**
  **Ichihara-shi**
  **Chiba 290-8551 (JP)**
- **YODA, Akiko**
  **Ichihara-shi**
  **Chiba 290-8551 (JP)**
- **SASAKI, Syuji**
  **Ichihara-shi**
  **Chiba 290-8551 (JP)**

(74) Representative: **Thurston, Joanna et al**
**Withers & Rogers LLP**
**4 More London Riverside**
**London SE1 2AU (GB)**

(56) References cited:
| | |
|---|---|
| EP-A1- 1 698 641 | WO-A1-00/48568 |
| JP-A- 09 067 266 | JP-A- 2000 178 196 |
| JP-A- 2003 012 489 | JP-A- 2003 238 399 |
| JP-A- 2003 300 824 | JP-A- 2006 274 245 |
| US-A- 5 609 723 | |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## EP 2 116 224 B1

**Description**

TECHNICAL FIELD

The present invention relates to a cosmetic composition comprising: one or more substances selected from sulfated cellulose and a salt thereof; and polyhydric alcohol.

BACKGROUND ART

In recent years, the number of people with sensitive skin, who are highly sensitive to chemical substances contained in cosmetics, has been increasing. People with sensitive skin tend to be highly sensitive to chemical substances contained in cosmetics because of skin roughness caused by drying. To that end, a humectant having a sufficient moisturizing effect and high safety has been desired in the cosmetic industries.

Human skin has hyaluronic acid, which has moisturizing ability, as well as hyaluronidase, which is a degrading enzyme for hyaluronic acid. When the balance between these two substances is lost and the amount of hyaluronic acid decreases; it tends to cause dryness and roughness of skin. It is thought that inflammation caused by hyaluronidase occurs based on the following mechanism: hyaluronic acid and glycosaminoglycan are degraded by hyaluronidase, and accordingly, cell surfaces or support matrix substances are destroyed. Then, cells are exposed and damaged by various substances such as pathogens, inflammation mediating substances, inflammatory agents and preservative agents. As a result, people with sensitive skin become highly sensitive to chemical substances contained in cosmetics.

Sulfated polysaccharides are known as compounds which inhibit the action of hyaluronidase and have moisturizing ability, i.e., hyaluronidase inhibitor compounds. It is thought that sulfated polysaccharides inhibit the activity of hyaluronidase to promote regeneration of cell surfaces and protective connection tissue matrices, thereby realizing anti-inflammatory action and tissue regeneration. Chondroitin sulfate is generally known as a typical example of sulfated polysaccharide. Further, sulfated cellulose, which has ability to inhibit hyaluronidase activity about 100 times that of chondroitin sulfate, is known (see Japanese Laid-Open Patent Publication -No. 2006-274245).

However, there is no example of cosmetic composition in which such a substance having high ability to inhibit hyaluronidase activity and moisturizing effect is blended. A cosmetic composition having a moisturizing effect and high ability to inhibit hyaluronidase activity, in which such a substance is blended, is desired.

WO 00/48568 A1 discloses a cosmetic composition comprising polyhydric alcohol and surfactants for use as a moisturiser.

REP 698 641 A1 discloses the use of sulfated cellulose salts as a hyaluronidase inhibitor in cosmetic compositions.

US 5,609,723 A1 discloses an oxygen bleeding system for a pulp in the presence of polyhydric alcohol.

JP 2006 274245A discloses the use of sulfated cellulose salts as a hydrolysis resisting compound and a skin anti-inflammatory agent.

JP 2000 178196 A discloses sulfated polysaccharides (cellulose) for use as a hyaluronidase inhibitor in cosmetic compositions.

DISCLOSURE OF THE INVENTION

One problem of the present invention is to provide a cosmetic composition, which has a moisturizing effect, and whose ability to inhibit hyaluronidase activity is high. Another problem of the present invention is to provide a cosmetic composition having the above-described effect and excellent chemical stability.

The present inventors diligently made researches in order to solve the above-described problems. As a result, the present inventors found that, by using one or more substances selected from sulfated cellulose and a salt thereof in combination with polyhydric alcohol, the ability of sulfated cellulose and the salt thereof to inhibit hyaluronidase activity is further improved, and that, by further blending an electrolyte having pH buffering ability, the obtained cosmetic composition has excellent stability. The present invention was achieved based on the above-described findings.

The present invention is constituted by the following matters:

(1) A cosmetic composition comprising: one or more substances selected from sulfated cellulose and a salt thereof (collectively referred to as "sulfated cellulose compounds"); and polyhydric alcohol.
(2) The cosmetic composition according to item (1), wherein the salt of sulfated cellulose is one or a combination of two or more of substances selected from lithium salt, potassium salt, sodium salt, beryllium salt, magnesium salt, calcium salt, triethylamine salt, arginine salt, lysine salt and histidine salt.
(3) The cosmetic composition according to item (1) or (2), comprising 0.001 to 20 wt% of one or more substances selected from the sulfated cellulose compounds and 0.0001 to 80 wt% of polyhydric alcohol.

(4) The cosmetic composition according to any one of items (1) to (3), wherein sulfated cellulose is obtained by sulfating crystalline cellulose having the average degree of polymerization of 100 to 300 using sulfuric anhydride or a complex of N,N-dimethylformamide and sulfuric anhydride.

(5) The cosmetic composition according to any one of items (1) to (4), wherein the sulfated cellulose compounds have: the weight average molecular weight of 1,000 to 200,000; the sulfur concentration of 6.5 to 19.0 wt%; and the solubility in pure water at 20°C of 3g/L or higher.

(6) The cosmetic composition according to any one of items (1) to (5), wherein polyhydric alcohol is one or a combination of two or more of substances selected from the group consisting of 1,2-ethanediol, diethylene glycol, triethylene glycol, polyethylene glycol, 1,2-propanediol, 1,3-propanediol, dipropylene glycol, polypropylene glycol, 1,2-butanediol, 1,3-butanediol, 1,4-butanediol, 3-methyl-1,3-butanediol, glycerin, 1,2-pentanediol, 1,3-pentanediol, 1,4-pentanediol, 1,5-pentanediol, 1,2,3-pentanetriol, 2,3,4-pentanetriol, 1,3,4-pentanetriol, 1,3,5-pentanetriol, 2-methyl-2,4-pentanediol, 1,2-hexanediol, 1,3-hexanediol, 1,4-hexanediol, 1,5-hexanediol, 1,6-hexanediol, 1,2,3-hexanetriol, 1,3,4-hexanetriol, 1,3,5-hexanetriol, 1,4,6-hexanetriol, erythritol, pentaerythritol, dipentaerythritol, mannitol, sorbitol, threitol, arabitol, xylitol, ribitol, galactitol, lactitol, maltitol, inositol, panthenol, cyclitol, laminitol, valienamine, validamine, validanol and 2-amino-2-methyl-1,3-propanediol.

(7) The cosmetic composition according to any one of items (1) to (6), comprising an electrolyte having pH buffering ability.

(8) The cosmetic composition according to item (7), wherein the content of the electrolyte having pH buffering ability in the cosmetic composition is 0.001 to 30 wt%.

(9) The cosmetic composition according to any one of items (1) to (8), wherein pH of the cosmetic composition is 5.5 to 7.0.

(10) The cosmetic composition according to any one of items (1) to (9), wherein the cosmetic composition is a hair treating agent.

(11) The cosmetic composition according to item (10), wherein the hair treating agent is a hair dressing (e.g., hair spray, hair tonic, hair gel, hair lotion, hair oil, hair essence, hair water, and hair wax), shampoo, finishing rinse, hair treatment, hair cream, hair mousse, hair setting lotion, hair color, hair dye (e.g., one-part hair dye, and two-part hair dye), perm solution (e.g., permanent wave solution, hair straightening solution, and permanent wave holding agent), hair growth tonic, pilatory or anti-hair loss agent.

(12) The cosmetic composition according to any one of items (1) to (9), wherein the cosmetic composition is a skin care cosmetic.

(13) The cosmetic composition according to item (12), wherein the skin care cosmetic is a toner, serum, whitening toner, milky lotion, whitening milky lotion, cream, whitening cream, ointment, whitening ointment, lotion, whitening lotion, oil or facial pack.

(14) The cosmetic composition according to any one of items (1) to (9), wherein the cosmetic composition is a makeup cosmetic.

(15) The cosmetic composition according to item (14), wherein the makeup cosmetic is a foundation, liquid foundation, lipstick, eye shadow, powder, blusher, eye liner, mascara or eyebrow pencil.

(16) The cosmetic composition according to any one of items (1) to (9), wherein the cosmetic composition is a skin cleanser.

(17) The cosmetic composition according to item (16), wherein the skin cleansers is a soap, cleansing cream, cleansing lotion, cleansing milk, facial wash or body shampoo.

(18) The cosmetic composition according to any one of items (1) to (9), wherein the cosmetic composition is a bath agent.

(19) The cosmetic composition according to any one of items (1) to (9), wherein the cosmetic composition is a finishing cosmetic (e.g., manicures).

(20) The cosmetic composition according to any one of items (1) to (9), wherein the cosmetic composition is a patch (or perfume, toothpaste or tooth wash).

The cosmetic composition of the present invention has a moisturizing effect, and its ability to inhibit hyaluronidase activity is high, and therefore, skin roughness caused by drying can be reduced. For this reason, the cosmetic composition of the present invention can be provided to people with sensitive skin, who are highly sensitive to chemical substances contained in cosmetics. The claims set form the composition according to the invention.

BEST MODE FOR CARRYING OUT THE INVENTION

Hereinafter, the present invention will be described in detail.

1. Sulfated cellulose

Sulfated cellulose to be used in the present invention is a compound, which is electrolyzed by sulfating a part or all of hydroxyl groups in cellulose, and to which water solubility is imparted. Sulfated cellulose of the present invention can be obtained by reacting cellulose with a sulfating agent to sulfate a part or all of hydroxyl groups in cellulose.

Cellulose, which is a raw material of sulfated cellulose to be used in the present invention, is not particularly limited as long as it is derived from plants, and publicly-known crosslinked or noncrosslinked celluloses can be used. In particular, crystalline cellulose is preferably used since it has high strength and is slightly soluble in water, thereby allowing inclusion of sulfur in sulfated cellulose at a high concentration. In particular, crystalline cellulose having the average degree of polymerization of 100 to 300 is preferably used, since sulfated cellulose which is chemically stable and excellent in hydrolysis resistance can be obtained thereby, and effects of a cosmetic composition is less likely to be decrease over time.

The sulfating agent to be reacted with cellulose is not particularly limited as long as it is generally used. However, because of more efficient reaction, sulfuric anhydride or a complex of N,N-dimethylformamide and sulfuric anhydride is preferred. In this regard, the complex of N,N-dimethylformamide and sulfuric anhydride is a compound which is formed by mixing N,N-dimethylformamide and sulfuric anhydride. The mixing ratio between N,N-dimethylformamide and sulfuric anhydride is preferably within a range in which there is an excess amount of N,N-dimethylformamide with respect to sulfuric anhydride, and the concentration of sulfuric anhydride in the mixture is more preferably 10 to 30 wt%. When the concentration of sulfuric anhydride is within this range, sulfation reaction of cellulose can be sufficiently progressed, and in addition, the sulfur concentration can be easily controlled. The use amount of the sulfating agent can be arbitrary selected depending on a desired sulfation ratio (or sulfur concentration) and reaction conditions, but the sulfating agent is suitably used in an amount of 1.2 to 3 equivalents per a hydroxyl group in cellulose.

In particular, in the cosmetic composition of the present invention, sulfated cellulose, which is obtained by sulfating crystalline cellulose having the average degree of polymerization of 100 to 300 using, as a sulfating agent, sulfuric anhydride or a complex of N,N-dimethylformamide and sulfuric anhydride, is preferably used.

The amount of sulfate ester (or sulfur concentration) in sulfated cellulose can be controlled by the feed ratio of the sulfating agent which may be reacted with hydroxyl groups relative to cellulose.

The solvent to be used for sulfation of cellulose is not particularly limited as long as the sulfating agent does not react therewith. Specific examples of the solvent include N,N-dimethylformamide, dimethylsulfoxide, heterocyclic solvents, such as dioxane and pyridine, and tertiary amine solvents such as triethylamine. The use amount of reaction solvent is 1 to 100 volumes, and preferably 1 to 10 volumes based on the weight of cellulose.

The reaction temperature is not particularly limited to a low temperature, and is preferably 10 to 25°C.

Sulfation reaction of cellulose is carried out by dissolving or suspending cellulose in a solvent, and thereafter dropping the sulfating agent therein. Dropping time of the sulfating agent is preferably 0.01 to 2 hours.

This reaction is a reaction between primary and secondary hydroxyl groups, and the sulfating agent. A reaction of primary hydroxyl groups progresses in a short time, but a reaction of secondary hydroxyl groups does not progress in a short time. Therefore, the reaction time is preferably 1 to 48 hours. Depending on the type of solvent used, there is a possibility that cellulose is not solved in a solvent. Therefore, it is preferred that stirring is carried out at a suitable rate for sufficient mixing. After the completion of reaction, an alcoholic solvent such as methanol and ethanol is added to the reaction solution to precipitate the obtained sulfated cellulose. Alternatively, the reaction may be terminated by addition of distilled water, followed by neutralization by alkali. The obtained mixture is filtrated or centrifuged to remove impurities, and the alcoholic solvent is dropped again on the filtrate to be crystallized, thereby obtaining sulfated cellulose.

2. Salt of sulfated cellulose

In the cosmetic composition of the present invention, as well as sulfated cellulose, a biologically acceptable salt of sulfated cellulose may also be used. Examples of such salts include salt with inorganic base, salt with organic base and salt with basic amino acid. Specific examples of salts with inorganic base include lithium salt, potassium salt, sodium salt, beryllium salt, magnesium salt, and calcium salt. Specific examples of salts with organic base include triethylamine salt. Specific examples of salts of basic amino acid include arginine salt, lysine salt and histidine salt. Further, the salts of sulfated cellulose to be used in the present invention include those in both anhydrous and hydrated forms.

In particular, sodium salt is preferred since it is easily produced, has high moisturizing effect and ability to inhibit hyaluronidase activity, and is relatively inexpensive.

These salts of sulfated cellulose can be obtained by reaction between sulfated cellulose and inorganic base, organic base or basic amino acid.

The weight average molecular weight of the sulfated cellulose compound to be used in the present invention is 1,000 to 200,000, and preferably 50,000 to 100,000. The sulfur concentration of the sulfated cellulose compound is 6.5 to 19.0 wt%, and preferably 12.0 to 17.0 wt%. The solubility of the sulfated cellulose compound in pure water at 20°C is 3 g/L or higher, preferably 10 g/L or higher, and more preferably 15 g/L or higher. The upper limit of the solubility is not particularly limited, but in general, it is preferably about less than 20 g/L. When the sulfated cellulose compound has any one or more of the above-described properties, a cosmetic compound obtained has an excellent moisturizing effect and ability to inhibit hyaluronidase activity, and therefore, skin roughness due to drying can be reduced. The weight average molecular weight of the sulfated cellulose compound can be obtained by the gel filtration method. The sulfur concentration of the sulfated cellulose compound refers to the content of sulfur per the dry weight of the sulfated cellulose compound, and it can be obtained by flask combustion - ion chromatography. The solubility of the sulfated cellulose compound in pure water at 20°C can be obtained by measuring the weight of the compound solved in 10 mL of pure water at 20°C.

3. Polyhydric alcohol

The polyhydric alcohol to be used in the present invention is not particularly limited as long as it is a compound having at least two hydroxyl groups linked to a linear, branched or cyclic hydrocarbon. Specific examples of the polyhydric alcohol include 1,2-ethanediol, diethylene glycol, triethylene glycol, polyethylene glycol, 1,2-propanediol, 1,3-propanediol, dipropylene glycol, 2-amino-2-methyl-1,3-propanediol, polypropylene glycol, 1,2-butanediol, 1,3-butanediol, 1,4-butanediol, 3-methyl-1,3-butanediol, glycerin, 1,2-pentanediol, 1,3-pentanediol, 1,4-pentanediol, 1,5-pentanediol, 1,2,3-pentanetriol, 2,3,4-pentanetriol, 1,3,4-pentanetriol, 1,3,5-pentanetriol, 1,2-hexanediol, 1,3-hexanediol, 1,4-hexanediol, 1,5-hexanediol, 1,6-hexanediol, 1,2,3-hexanetriol, 1,3,4-hexanetriol, 1,3,5-hexanetriol, 1,4,6-hexanetriol, 2-methyl-2,4-pentanediol, erythritol, pentaerythritol, dipentaerythritol, mannitol, sorbitol, threitol, arabitol, xylitol, ribitol, galactitol, lactitol, maltitol, inositol, panthenol, cycilitol, laminitol, valienamine, validamine, validatol, ethylene oxide, ethyleneglycol monoethylether, ethyleneglycol monobutylether, diethylene glycolmonomethylether, diethylene glycolmonoethylether and propylene oxide. In particular, 1,3-butanediol, 1,2-propanediol, polyethylene glycol, 1,2-pentanediol, 1,2-hexanediol and sorbitol, are preferred. In the present invention, the polyhydric alcohol may equally be used in both anhydrous and hydrated forms.
The polyhydric alcohol to be used in the present invention may be prepared using any one of various known techniques such as organic synthesis, extraction and purification from a naturally-occurring product, or fermentation by microorganisms. Polyhydric alcohols obtained thereby may be used without particular limitation.
Any of the forms may be used for the polyhydric alcohol in the present invention, such as fine powders, microcrystals, liquids, pellets, viscous fluids or gels, for example. The most suitable form for cosmetic composition products of the present invention may be selected.

4. Cosmetic composition

The cosmetic composition of the present invention contains: one or more substances selected from the sulfated cellulose compounds; and polyhydric alcohol as essential components. By using the sulfated cellulose compound in combination with polyhydric alcohol, the cosmetic composition of the present invention retains a suitable concentration of the sulfated cellulose compound and exerts an excellent moisturizing effect and ability to inhibit hyaluronidase activity.
The content of the sulfated cellulose compound in the cosmetic composition of the present invention is not particularly limited, but in terms of the cost of raw materials for products and the concentration range in which a desired moisturizing effect and effect of inhibiting hyaluronidase activity can be exerted, the content is arbitrary set within a range of preferably 0.0001 to 50 wt%, and more preferably 0.001 to 1 wt%.
The content of polyhydric alcohol in the cosmetic composition of the present invention is not particularly limited, but in terms of the cost of raw materials for products and the concentration range in which a desired moisturizing effect can be exerted, the content is arbitrary set within a range of preferably 0.0001 to 80 wt%, and more preferably 0.001 to 50 wt%.
The preferable range of the content ratio between the sulfated cellulose compound and polyhydric alcohol in the cosmetic composition of the present invention slightly varies depending on the form of the cosmetic composition. For example, when using the cosmetic composition of the present invention as a hair treating agent, the content of the sulfated cellulose compound is preferably 0.01 to 5 wt%, and more preferably 0.1 to 1 wt%, and the content of polyhydric alcohol is preferably 0.1 to 80 wt%, and more preferably 0.5 to 15 wt%.
When using the cosmetic composition of the present invention as a skin care cosmetic, the content of the sulfated cellulose compound is preferably 0.01 to 5 wt%, and more preferably 0.05 to 1 wt%, and the content of polyhydric alcohol is preferably 1 to 20 wt%, and more preferably 3 to 10 wt%.
When using the cosmetic composition of the present invention as a makeup cosmetic, the content of the sulfated cellulose compound is preferably 0.01 to 5.0 wt%, and more preferably 0.1 to 1 wt%, and the content of polyhydric alcohol is preferably 0.5 to 20 wt%, and more preferably 1 to 10 wt%.

When using the cosmetic composition of the present invention as a skin cleanser, the content of the sulfated cellulose compound is preferably 0.01 to 5 wt%, and more preferably 0.1 to 1 wt%, and the content of polyhydric alcohol is preferably 1 to 30 wt%, and more preferably 5 to 15 wt%.

When using the cosmetic composition of the present invention as a bath agent, the content of the sulfated cellulose compound is preferably 0.01 to 10 wt%, and more preferably 0.1 to 1 wt%, and the content of polyhydric alcohol is preferably 1 to 10 wt%, and more preferably 1 to 5 wt%.

When using the cosmetic composition of the present invention as a patch, the content of the sulfated cellulose compound is preferably 0.01 to 5 wt%, and more preferably 0.1 to 1 wt%, and the content of polyhydric alcohol is preferably 5 to 30 wt%, and more preferably 5 to 20 wt%.

By using the sulfated cellulose compound in combination with polyhydric alcohol, the cosmetic composition of the present invention can exert an excellent moisturizing effect, but a sulfate ester group contained in sulfated cellulose may generally be a reactive spot of hydrolysis reaction under acidic conditions. In particular, under strongly-acidic conditions, there is a possibility that hydrolysis reaction is caused at an accelerated rate. For this reason, in addition to the sulfated cellulose compound and polyhydric alcohol, an electrolyte having pH buffering ability is preferably contained in the cosmetic composition of the present invention. The electrolyte having pH buffering ability to be used in the present invention is not particularly limited, but is preferably a combination of weak acid and weak acid salt.

Specific examples of the electrolyte having pH buffering ability to be used in the present invention include phosphoric acid-metal salt of phosphoric acid, acetic acid-metal salt of acetic acid, lactic acid-metal salt of lactic acid, succinic acid-metal salt solution of succinic acid, tartaric acid-metal salt of tartaric acid, citric acid-metal salt of citric acid, and malic acid-metal salt of malic acid. The type of metal salt is not particularly limited, but in terms of the solubility, sodium salt or potassium salt is preferred.

The content of the electrolyte having pH buffering ability (for example, when using a combination of weak acid and weak acid salt, the total amount thereof) in the cosmetic composition of the present invention is not particularly limited, but because of exertion of sufficient buffering effect, the content is arbitrary set within a range of preferably 0.001 to 30 wt%, and more preferably 0.01 to 10 wt%.

pH of the cosmetic composition of the present invention is arbitrary set within a range of preferably 5.5 to 7.0, more preferably 5.5 to 6.5, and even more preferably 5.5 to 6.2.

Within the above-described range, problems of skin redness, skin irritation and the like at the time of use of the cosmetic composition can be prevented, and accordingly, the cosmetic, composition having excellent safety can be provided.

The cosmetic composition of the present invention may be selected depending on a specific product formation of the cosmetic composition from fine powders, microcrystals, liquids, or pellets.

The cosmetic composition of the present invention may be formed into granules, gels, or viscous fluids by mixing it with, for example, an excipient, thickened or gelling agent.

The cosmetic composition of the present invention may be formulated in various forms such as a capsule, powder, granule, solid, liquid, gel, foam, emulsion, cream, ointment, sheet, mousse, powder dispersion, multilayer, and aerosol.

The cosmetic composition of the present invention finds applications in hair treating agents such as hair dressings (e.g., hair sprays, hair tonics, hair gels, hair lotions, hair oils, hair essences, hair waters, and hair waxes), shampoos, finishing rinses, hair treatments, hair creams, hair mousses, hair setting lotions, hair colors, hair dyes (e.g., one-part hair dyes and two-part hair dyes), perm solutions (e.g., permanent wave solutions, hair straightening solutions, and permanent wave holding agents), hair growth tonics, pilatories, and anti-hair loss agents. Another application of the composition includes, for example, skin care cosmetics such as toners, serums, whitening toners, milky lotions, whitening milky lotions, creams, whitening creams, ointments, whitening ointments, lotions, whitening lotions, oils, and facial packs. Furthermore, still another application of the composition according to the present invention includes, for example, makeup cosmetics such as foundations, liquid foundations, lipsticks, eye shadows, powders, blushers, eye liners, mascaras, and eyebrow pencils. Another application of the composition according to the present invention includes, for example, skin cleansers such as soap, cleansing creams, cleansing lotions, cleansing milks, facial washes, and body shampoos. Moreover, another application of the composition according to the present invention includes finishing cosmetics such as manicures. Other applications of the composition according to the present invention include, for example, bath agents, patches, perfumes, toothpastes, and tooth washes.

5. Additives for the cosmetic composition

In addition to the essential ingredients, the sulfated cellulose compound and polyhydric alcohol, the cosmetic composition of the present invention may contain the following exemplary ingredients that are arbitrarily selected and combined as additives to provide a cosmetic composition having a larger variety of functions: active ingredients such as pigmentation inhibitors, tyrosinase inhibitors, melanocyte melanogenesis inhibitors, melanogenesis promoters, humectants, cell activators/metabolic activators, antioxidants, active oxygen scavengers/radical production inhibitors, lipometabolism promoters, UV blockers/UV absorption promoters, astringents, anti-inflammatory agents/interleukin inhibitors/antiphlogistics, antiseborrheic agents, antimicrobial agents/antiviral agents, blood flow enhancers/blood vessel stimulators, anti-androgen agents, structural proteolytic enzyme (e.g., elastase, collagenase, keratin protease, serine protease, integrin degrading enzyme, involucrin degrading enzyme, filaggrin degrading enzyme, laminin degrading enzyme, fibronectin degrading enzyme, and proteoglycan degrading enzyme) inhibitors, structural protein synthesis promoters, mucopolysaccharides (e.g., hyaluronic acid, and chondroitin sulfuric acid) degrading enzyme inhibitors, mucopolysaccharides synthesis promoters, intercellular lipid production promoters/intercellular lipid condition modifiers, keratolytic agents/stratum corneum removal promoters, plasminogen-activator competitive inhibitors, Maillard reaction inhibitors, testosterone 5 alpha-reductase inhibitor/hair papilla activating agents/hair growth promoters, hair matrix cell proliferation inhibitors/hair growth inhibitors, hair swelling agents/hair protectors, and odor counteractants, and other plant-based materials, animal-based materials, microorganism-based materials, extracts and metabolites originated from naturally-occurring materials or various chemical compounds that are preferably used for preparation of cosmetic compositions.

The content in the preparation is not specifically limited but it may generally be within the concentration range of from 0.0001 to 50 wt%.

(1) Pigmentation inhibitors

The cosmetic composition of the present invention may contain a pigmentation inhibitor. Specific examples of the pigmentation inhibitor include p-aminobenzoic acid derivatives, salicylic acid derivatives, benzenesulfonamide derivatives, imidazole derivatives, naphthalene derivatives, hydroxyanthranilic acid or salts thereof and their derivatives, anthranilic acid derivatives, coumarin derivatives, amino acid derivatives, benzotriazole derivatives, tetrazole derivatives, imidazoline derivatives, pyrimidine derivatives, and dioxane derivatives.

(2) Tyrosinase inhibitor

The cosmetic composition of the present invention may contain a tyrosinase inhibitor. Specific examples of the tyrosinase inhibitor include ascorbic acid or salts thereof and their derivatives, hydroquinone or salts thereof and their derivatives, kojic acid or salts thereof and their derivatives, tocopherol or salts thereof and their derivatives, tocotrienol or salts thereof and their derivatives, N-acetyl tyrosine or salts thereof and their derivatives, glutathione or salts thereof and their derivatives, and ellagic acid or salts thereof and their derivatives.

(3) Melanocyte melanogenesis inhibitor

The cosmetic composition of the present invention may contain a melanocyte melanogenesis inhibitor. Specific examples of the melanocyte melanogenesis inhibitor include lobeline or lobeline derivatives, liquiritin derivatives, phenylchroman derivatives, chromone derivatives, azelaic acid derivatives, phosphatidylglucosamine, lysophosphatidylglucosamine, phenylhydroquinone, 3-beta-D-glucopyranosyl manool, 3-beta-D-maltopyranosyl manool, substituted amino acid derivatives, benzolactam derivatives, and indolactam derivatives.

(4) Melanogenesis promoter

The cosmetic composition of the present invention may contain a melanogenesis promoter. Specific examples of the melanogenesis promoter include salicylic acid or salts thereof and their derivatives, salicyl alcohol or salts thereof and their derivatives, apigenin, amentoflavone, *Zanthoxylum piperitum* extract, *Aralia cordata* extract, *Angelica pubescens* extract, *Anemone flaccida* extract, kashi extract, *Circaea cordata* extract, and *Ageratum conyzoides* L. extract.

(5) Humectant

The cosmetic composition of the present invention may contain a humectant. Specific examples of the humectant include gum arabic, benzoin gum, dammar gum, guaiac gum, Irish moss, karaya gum, tragacanth gum, carob gum, quince seed, agar or derivatives thereof, casein, glucose, galactose, mannose, xylose, fructose, maltose, isomaltose, cellobiose, gentiobiose, and trehalose.

(6) Cell activator/Metabolic activator

The cosmetic composition of the present invention may contain a cell activator/metabolic activator. Specific examples of the cell activator/metabolic activator include vitamin A group: retinol or salts thereof and their derivatives, retinal or salts thereof and their derivatives, dehydroretinal or salts thereof and their derivatives, and retinoic acid or salts thereof and their derivatives.

(7) Antioxidant

The cosmetic composition of the present invention may contain an antioxidant. Specific examples of the antioxidant include ascorbic acid or salts thereof and their derivatives, stearic acid ester, tocopherol or salts thereof and their derivatives, dihydropyridine derivatives, benzochroman derivatives, norujihidoroguasereten acid, butylhydroxytoluene (BHT), and butylhydroxyanisole (BHA).

(8) Active oxygen scavenger/Radical scavenger

The cosmetic composition of the present invention may contain an active oxygen scavenger/radical scavenger. Specifics examples of the active oxygen scavenger/radical scavenger include superoxide dismutase, catalase, glutathione peroxidase, bilirubin, quercetin, quercitrin, catechin, catechin derivatives, rutin or derivatives thereof, gallic acid or salts thereof and their derivatives, and curcumin or salts thereof and their derivatives.

(9) Lipometabolism promoter

The cosmetic composition of the present invention may contain a lipometabolism promoter. Specific examples of the lipometabolism promoter include phthalazine derivatives, *Cocculus trilobus* (MU FANG JI) extract, *Cirsium japonicum* extract, *Cephalonoplos segetum* extract, *Cirsium borealinipponense* extract, *Cirsium maritimum* extract, *Cirsium japonicum* extract, *Rhaponticum uniflorum* extract, *Theobroma cacao* extract, *Gentianella alborosea* extract, *Sinomenium acutum* (FAN JI) extract, *Curcuma zedoaria* (E ZHU) extract, *Fumaria officinalis* extract, and *Platycodon grandiflorum* (JIE GENG, JIE GENG GEN) extract.

(10) UV blocker/UV absorption promoter

The cosmetic composition of the present invention may contain a UV blocker/UV absorption promoter. Specific examples of the UV blocker/UV absorption promoter include benzophenone derivatives, 1,2-dihydroxy-4-(2-hydroxyethyl)benzene derivatives, paraaminobenzoic acid derivatives, methoxycinnamic acid derivatives, anthranilic acid derivatives, urocanic acid derivatives, coumarin derivatives, amino acid-based compounds, benzotriazole derivatives, and tetrazole derivatives.

(11) Astringent

The cosmetic composition of the present invention may contain an astringent. Specific examples of the astringent include succinic acid, allantoin, zinc chloride, zinc sulfate, zinc oxide, calamine, zinc p-phenolsulfonate, aluminum potassium sulfate, resorcin, ferric chloride, and tannins.

(12) Anti-inflammatory agent/interleukin inhibitor/histamine release inhibitor

The cosmetic composition of the present invention may contain an anti-inflammatory agent/interleukin inhibitor/histamine release inhibitor. Specific examples of the anti-inflammatory agent/interleukin inhibitor/histamine release inhibitor include quinolinone derivatives, dibenzoxepin derivatives, thiotropocin, phthalimide derivatives, flurbiprofen, felbinac, bufexamac, suprofen, 1,4-diphenylpropylpiperazine derivatives, and calyxin compounds.

(13) Antiseborrheic agent

The cosmetic composition of the present invention may contain an antiseborrheic agent. Specific examples of the antiseborrheic agent include chroman derivatives, pyridoxine or salts thereof and their derivatives, pyridoxal or salts thereof and their derivatives, pyridoxiamine or salts thereof and their derivatives, sulfur, *Cannabis sativa* (HUO MA REN) extract, *Lamium album var. barbatum* (ZOKUDAN) extract, *Nasturtium officinale* (cresson) extract, *Valeriana fauriei* (KISSOUKON) extract, and Clematis extract.

(14) Antimicrobial agent

The cosmetic composition of the present invention may contain an antimicrobial agent. Specific examples of the antimicrobial agent include acrinol, sulfur, gluconic acidcalcium, chlorhexidine gluconate, sulfamin, mercurochrome, lactoferrin or hydrolysates thereof, alkyldiaminoethylglycine chloride solution, triclosan, sodium hypochlorite, chloramine T, calcium hypochlorite, iodine compounds, and iodoform.

(15) Blood flow enhancer/blood vessel stimulator

The cosmetic composition of the present invention may contain a blood flow enhancer/blood vessel stimulator. Specific examples of the blood flow enhancer/blood vessel stimulator include tocopherol or salts thereof and their derivatives, tocotrienol or salts thereof and their derivatives, cepharanthine, carpronium chloride, eugenol derivatives, minoxidil, capsicum tincture, and nonylic vanillylamide.

(16) Anti-androgen agent

The cosmetic composition of the present invention may contain an anti-androgen agent. Specific examples of the anti-androgen agent include follicular hormone, isoflavone, oxendolone, 4',5,7-trihydroxy-8-prenylflavanone, 4',5,7-trihydroxy-8-prenylflavone, 3,3',4',5,7-pentahydroxy-8-prenylflavone, nicorandil, and cyclosporine acid.

(17) Structural proteolytic enzyme inhibitor/structural proteolytic enzyme expression inhibitor

The cosmetic composition of the present invention may contain a structural proteolytic enzyme (e.g., a matrix metalloproteinase such as elastase, collagenase, keratin protease, serine protease, integrin degrading enzyme, involucrin degrading enzyme, filaggrin degrading enzyme, laminin degrading enzyme, fibronectin degrading enzyme, proteoglycan degrading enzyme) inhibitor/structural proteolytic enzyme expression inhibitor. Specific examples of the structural proteolytic enzyme inhibitor/structural proteolytic enzyme expression inhibitor include adenine derivatives, carbostyril derivatives or salts thereof, dicarboxylic acids, rosmarinic acid, ursolic acid, oleanolic acid, hydroxamic acid derivatives, esculetin derivatives, anthocyanidins, and nordihydroguaiaretic acid.

(18) Structural protein synthesis promoter

The cosmetic composition of the present invention may contain a structural protein synthesis promoter. Specific examples of the structural protein synthesis promoter include ethanolamine derivatives, pentoxifylline, serine derivatives, geraniol, crocetin, methyl 4-(2-ethylhexyloxy)-2-hydroxybenzoate, methyl 2-hydroxy-4-(3,5,5-trimethylhexyloxy)benzoate, methyl 4-cyclohexylmethoxy-2-hydroxybenzoate, ethyl 4-(2-cyclohexylethoxy)-2-hydroxybenzoae, and methyl 4-(3,7-dimethyl-6-octenyloxy)-2-hydroxybenzoate.

(19) Mucopolysaccharides degrading enzyme inhibitor

The cosmetic composition of the present invention may contain mucopolysaccharides (e.g., hyaluronic acid, and chondroitin sulfuric acid) degrading enzyme inhibitor. Specific examples of the mucopolysaccharides degrading enzyme inhibitor include anacardic acid or derivatives thereof, isoprenylated benzophenone derivatives, *Epimedium grandiflorum var. thunbergianum* extract, *Epimedium grandiflorum Morr. var. Grandiflorum* (YIN YANG HUO) extract, *Polygonatum falcatum* extract, *Polygonatum sibiricum* (HUANG JING) extract, *Origanum rotundifolium* extract, *Dryopteridaceae* extract, *Polygala tenuifolia* extract, and *Prunus jamasakura* extract.

(20) Mucopolysaccharides synthesis promoter

The cosmetic composition of the present invention may contain mucopolysaccharides synthesis promoter. Specific examples of the mucopolysaccharides synthesis promoter include stilbene derivatives or salts thereof, mollugin or salts thereof and their derivatives, N-acetylglucosamine, *Linum usitatissimum* extract, *Broussonetia papyrifera* (CHOJITSU) extract, *Broussonetia kazinoki* extract, *Origanum majorana L.* extract, *Artocarpus altilis* extract, and Hirakotoji extract.

(21) Intercellular lipid production promoter/intercellular lipid condition modifier

The cosmetic composition of the present invention may contain an intercellular lipid production promoter/intercellular lipid condition modifier. Specific examples of the intercellular lipid production promoter/intercellular lipid condition modifier include phospholipids, sterins, N-acetylneuraminic acid, N-glucosol neuraminic acid, gangliosides, oligosulfated hyaluronic acid, hydroxytamoxifen compounds, glycoglycerolipids, pentoxifylline, and 3-deazaadenosine.

(22) Keratolytic agent/stratum corneum removal promoter

The cosmetic composition of the present invention may contain a keratolytic agent/stratum corneum removal promoter. Specific examples of the keratolytic agent/stratum corneum removal promoter include tropolone and derivatives thereof, resorcin, lactic acid, urea, salicylic acid, guanidine, ethanolamine, *Trichosanthes kirilowii var. japonica* (KAROKON) extract, *Trigonella foenum-graecum* extract, *Lablab purpureus* (BIAN DOU) extract, *Lens esculenta* extract, *Cicer arietinum* extract, and *Vigna radiata* extract.

(23) Plasminogen-activator competitive inhibitor

The cosmetic composition of the present invention may contain a plasminogen-activator competitive inhibitor. Specific examples of the plasminogen-activator competitive inhibitor include *Arnica unalaschkensis var. tschonoskyi* extract, *Humulus japonicus* extract, *Humulus lupulus var. cordifolius* extract, and *Rubus idaeus* extract.

(24) Maillard reaction inhibitor

The cosmetic composition of the present invention may contain a Maillard reaction inhibitor. Specific examples of the Maillard reaction inhibitor include aminoguanidine, flavanones, 2-hydroxyphenylalkylamine derivatives or salts thereof, phenylpropenoic acid derivatives, citric acid or salts thereof, *Acorus gramineus* (SHI CHANG GEN) extract, *Bletilla striata* extract, *Mallotus japonicus* extract, *Diospyros kaki* (including SHI DI) extract, and *Morus alba* extract.

(25) Testosterone 5 alpha-reductase inhibitor/hair papilla activating agent/hair growth promoter

The cosmetic composition of the present invention may contain testosterone 5 alpha-reductase inhibitor/hair papilla activating agent/hair growth promoter. Specific examples of the testosterone 5 alpha-reductase inhibitor/hair papilla activating agent/hair growth promoter include gamma-amino-beta-hydroxybutyric acid esters, amineoxides, alkylbetaines, pyrimidine-N-oxide derivatives, p-menthane-3,8-diol, monoglyceryl-D-glucoside monotridecanoate, 1-o-N-pentadecylglycero-D-glucoside, and glyceride sulfates such as monopentadecanoic acid glyceride sulfuric acid ester salt.

(26) Hair matrix cell proliferation inhibitor/hair growth inhibitor

The cosmetic composition of the present invention may contain a hair matrix cell proliferation inhibitor/hair growth inhibitor. Specific examples of the hair matrix cell proliferation inhibitor/hair growth inhibitor include phthalazinones, benzoxazinones, phosphonic acid derivatives, cyproterone, 5-alpha-androstene-3alpha,17beta-diol, medroxyprogesterone, norethisterone, mestanolone, *Iris tectorum* (Ichihatsu) extract, HE SHOU WU *(Polygonum multiflorum* radix) extract, Kantou extract, *Cremastra appendiculata* extract, *Gracilaria bursa-pastoris* extract, CANG ZHU (*Atractylodes lancea* rhizome) extract, *Genista tinctoria* extract, FU PING CAO (*Spirodelapolyrrhiza* leaf) extract, myrrh (*Commiphora myrrha*) extract, *Champia parvula* extract, and *Sargassum siliquastrum* extract.

(27) Hair swelling agent/hair protector

The cosmetic composition of the present invention may contain a hair swelling agent/hair protector. Specific examples of the hair swelling agent/hair protector include ethanolamine, urea, guanidine, silicones, blueberry (*Vaccinium corymbosum*) extract, and mango extract.

(28) Odor counteractant

The cosmetic composition of the present invention may contain an odor counteractant. Specific examples of the odor counteractant include *Anethum graveolens* extract, Elemi (*Canarium luzonicum*) extract, dammar (*Agathis dammara*) extract, vanilla beans extract, and *Pinus thunbergii* extract.

Additional specific examples of the above-described components to be used in the cosmetic composition of the present invention are described in Japanese Laid-Open Patent Publication No. 2005-350454 and EP Laid-Open Publication No. 1604647, and therefore these publications can be referred to.

6. Ingredients used as additives

Specific examples of ingredients derived from a plant source, which are used by being added to the cosmetic composition of the present invention as additives, include plant extracts such as *Sabia japonica* (QING FENG TENG) extract, *Gastrodia elata Blume f. viridis* extract, *Ribes sativum* extract, *Ulmus parvifolia* (ROUYUPI) extract, aguai guasu extract, abiu extract, abiurana-abiu extract, yellow sapote extract, *Cananga odorata* extract, and *Orchis graminifolia* extract.

Specific examples of ingredients derived from a plant source, which are used by being added to the cosmetic composition of the present invention as additives, include *Dunaliella* sp. extract, *Chlorococcus* sp. extract, *Pandorina morum* extract, *Volvox aureus* extract, *Volvox* sp. extract, *Palmella* extract, *Tetraspora* extract, *Spirogyra* sp. and *Mougeotia* sp. extract, *Draparnaldia* extract, and *Ulothrix zonata* extract.

Specific examples of ingredients derived from an animal source, which are used by being added to the cosmetic composition of the present invention as additives, include cockscomb extract, placenta extract derived from bovine placenta, swine placenta or human placenta, extract derived from swine or bovine stomach, duodenum, intestine, or spleen or degradation products thereof, extract derived from bovine or swine brain tissue, collagen derivatives such as water-soluble collagen and acylated collagen, and collagen hydrolysate.

Specific examples of ingredients derived from a microorganism source, which are used by being added to the cosmetic composition of the present invention as additives, include yeast metabolites, yeast extracts, bacteria metabolites, bacteria extract, metabolites of mold or mushroom, actinomycete metabolite, extract from mold or mushroom, actinomycete extract, Bacillus natto metabolite, Bacillus natto extract, rice fermented extract, rice bran (red bran, white bran) fermented extract, euglena extract or degradation products thereof or their water-soluble derivatives, lactic fermented raw milk or nonfat dry milk product, and trehalose or derivatives thereof.

Materials derived from a plant or animal or microorganism source may comprise any sites, cells, tissues, organs, and metabolites derived from a transgenic lines or cell fusion products. In addition, tissue-derived cultured cells (e.g., cultured cells derived from animals, such as fibroblasts, Langerhans cells, macrophages, epidermal cells, and liver cells), undifferentiated cells, cells under differentiation processes, and metabolites thereof, which may be obtained by cultivation of sites, cells, tissues or organs, for example may be used.

Specific examples of naturally-occurring ingredients which may be used as additives include sea water such as deep water, e.g., sea water salt, dried sea water, inorganic salts obtained from the Dead Sea or Atlantic Ocean or Pacific Ocean (e.g., sodium chloride, magnesium chloride, and potassium chloride), and sea mud or fango.

Additional specific examples of the above-described additives to be used in the cosmetic composition of the present invention are described in Japanese Laid-Open Patent Publication No. 2005-350454 and EP Laid-Open Publication No. 1604647, and therefore these publications can be referred to.

Extracts derived from a plant or animal or microorganism source and other extracts derived from naturally-occurring materials, which are used as additives, may be obtained by carrying out standard procedures (for example, an appropriate combination of grinding, milling, washing, extraction, degradation, fermentation or metabolic shifting by microorganisms, fractionation, purification, compression, filtration, drying, pulverization, granulation, dissolution, sterilization, pH adjustment, deodorization, and bleaching) depending on the type and formation of the product to which they are to be added, and then performing selection from any of the resulting materials. These procedures are also described in the above-described publications.

7. Other compounds to be used as additives for the cosmetic composition of the present invention

Specific examples of other compounds which are used as additives for the cosmetic composition of the present invention include inorganic pigments, inorganic reducing agents, oxidizing reducing agents, dyes, fragrances including: natural animal essences such as musk, civet, castoreum, and ambergris; botanical fragrances; and other synthetic fragrances, pigments and coloring agents, fats and oils, waxes, mineral oils, fatty acids including natural fatty acids and synthetic fatty acids, alcohols, esters, metal soaps, anionic surfactants, sulfated oils, anionic surfactants, cationic surfactants, nonionic surfactants, amphoteric surfactants, natural surfactants, amino acid surfactants, silicone surfactants, silicone compounds, and hair treatment agents. Additional specific examples of the above-described additives are described in Japanese Laid-Open Patent Publication No. 2005-350454 and EP Laid-Open Publication No. 1604647, and therefore these publications can be referred to.

The following additives may also be used in combination in the present invention: hormones, sequestrants, pH adjusting agents, chelating agents, antiseptic/antifungal agents, agents capable of generating a cool sensation, stabilizers, emulsifiers, animal and plant proteins and degradation products thereof, animal and plant polysaccharides and degradation products thereof, animal and plant glycoproteins and degradation products thereof, antiphlogistic/antiallergic agents, wound healing agents, foam boosters, thickeners, enzymes, purified waters (e.g., electronic water, small clustering), deodorants/deodorization agents.

The cosmetic composition of the present invention is produced using a method in which one or more substances selected from the sulfated cellulose compounds, polyhydric alcohol, and if required, the above-described various additives are weighed independently to have a ratio suitable to an application, and thereafter these materials are mixed together. Publicly-known production apparatuses and production conditions may be employed depending on the properties and application of the cosmetic composition.

For example, in the case of hair dressing, it may be prepared, for example, by dissolving POE hydrogenated castor oil or fragrance, in a mixture of ethanol, 1,3-butanediol, etc., adding to the mixture purified water in which the sulfated cellulose compounds, a UV blocker or a preservative agent, are dissolved, and stirring the mixture homogeneously. However, the production method is not limited thereto.

In the case of shampoo, it may be prepared, for example, by mixing lauryldimethylamine oxide with a cationic compound such as stearyl dimethylamine, adding to the mixture purified water in which the sulfated cellulose compounds, propylene glycol, carboxyvinyl polymer or a pH adjusting agent, are dissolved to be emulsified, and stirring the mixture homogeneously. However, the production method is not limited thereto.

In the case of finishing rinse, hair treatment, hair cream, for example, it may be prepared, for example, by adding polyhydric alcohol such as 1,2-pentanediol to a mixture of tertiary ammonium chloride or isopropyl palmitate, adding to the mixture purified water in which the sulfated cellulose compounds, a pH adjusting agent, are dissolved to be emulsified, and stirring the mixture homogeneously. However, the production method is not limited thereto.

In the case of hair color, hair dye, perm solution, for example, for example, a first solution may be prepared by mixing a basic organic compound such as diaminotoluene sulfate salt with ammonia water, adding to the mixture alcohols such as oleyl alcohol and 1,2-propanediol, adding to the mixture purified water in which the sulfated cellulose compounds or sodium sulfite, are dissolved, and stirring the mixture homogeneously. A second solution may be prepared by adding purified water in which fragrance, for example is dissolved to a mixture of hydrogen peroxide water, 1,3-butanediol for example, and stirring the mixture homogeneously. However, the production method is not limited thereto.

In the case of hair growth tonic or pilatory, for example, it may be prepared, for example, by dissolving capsicum tincture, or tocopherol acetate, for example in ethanol or 1,5-pentanediol for example, adding to the mixture purified water in which the sulfated cellulose compounds, sodium glutamate, or resorcin, for example are dissolved, and stirring the mixture homogeneously. However, the production method is not limited thereto.

In the case of toner, it may be prepared, for example, by dissolving POE hydrogenated castor oil, or fragrance, in a mixture of ethanol and isoprene glycol or the like, adding to the mixture purified water in which the sulfated cellulose compounds, a UV blocker, a preservative agent, etc. are dissolved, further adding a pH adjusting agent thereto, and stirring the mixture homogeneously. However, the production method is not limited thereto.

In the case of serum, it may be prepared, for example, by dissolving sorbitan sesquiisostearate, or decamethylcyclopentasiloxane, in alcohols such as 1,2-propanediol, and adding to the mixture purified water in which the sulfated cellulose compounds, or a pH adjusting agent, are dissolved. However, the production method is not limited thereto.

In the case of milky lotion, it may be prepared, for example, by mixing olive oil, liquid paraffin, POE oleyl ether, sorbitan sesquioleate, or a preservative agent, adding to the mixture purified water in which the sulfated cellulose compounds, hyaluronic acid, propylene glycol, carboxy vinyl polymer or pH adjusting agent, are dissolved to be emulsified, adding to the obtained mixture ethanol, a UV blocker, pigments, or fragrance, and stirring the mixture homogeneously. However, the production method is not limited thereto.

In the case of cream, it may be prepared, for example, by mixing cetyl alcohol, stearyl alcohol, stearic acid, liquid paraffin, petrolatum, sorbitan monostearate, POE monostearate, a preservative agent, etc., adding to the mixture purified water in which the sulfated cellulose compounds, 1,3-butylene glycol or a pH adjusting agent, are dissolved to be emulsified, cooling the mixture and thereafter further adding dipotassium glycyrrhizinate, a UV blocker or pigment, fragrance, and stirring the mixture homogeneously. However, the production method is not limited thereto.

In the case of foundation, it may be prepared, for example, by dissolving dipropylene glycol, in decamethylcyclopentasiloxane, mixing the mixture with an inorganic compound such as hydrophobic treated titanium oxide and hydrophobic treated talc, adding to the mixture purified water in which the sulfated cellulose compounds, fragrance, are dissolved, and stirring the mixture homogeneously. However, the production method is not limited thereto.

In the case of liquid foundation, it may be prepared, for example, by dissolving ethanol, in decamethylcyclopentasiloxane, mixing the mixture with hydrophobic treated titanium oxide or spherical resin powder, adding to the mixture purified water in which the sulfated cellulose compounds or fragrance, are dissolved, and stirring the mixture homogeneously. However, the production method is not limited thereto.

In the case of lipstick, it may be prepared, for example, by dissolving ceresin wax or cadelin wax, in decamethylcyclopentasiloxane, mixing the mixture with the sulfated cellulose compounds, propylene glycol dicaprate or polyglyceryl diisostearate, and thereafter adding pigment such as Red No. 201 or hydrophobic treated mica-titanium, thereto and stirring the mixture homogeneously. However, the production method is not limited thereto.

In the case of eye shadow, it may be prepared, for example, by mixing hydrophobic-treated chromium oxide, ultramarine or titanium-coated mica, with decamethylcyclopentasiloxane, adding polyhydric alcohols such as propylene glycol thereto, adding to the mixture purified water in which the sulfated cellulose compounds or fragrance, are dissolved, and stirring the mixture homogeneously. However, the production method is not limited thereto.

In the case of facial wash, it may be prepared, for example, by mixing glycerin with triethanolamine lauryl phosphate, adding to the mixture purified water in which the sulfated cellulose compounds or auryl hydroxysulfobetaine, and fragrance are dissolved, and stirring the mixture homogeneously. However, the production method is not limited thereto.

In the case of body shampoo, it may be prepared, for example, by mixing fatty acid salts such as potassium laurate and potassium myristate with 1,2-propanediol or amino-modified aminomethylpolysiloxane, adding to the mixture purified water in which the sulfated cellulose compounds or fragrance, are dissolved, and stirring the mixture homogeneously. However, the production method is not limited thereto.

In the case of bath agent, it may be prepared, for example, by mixing sodium hydrogen carbonate with sodium borate, adding to the mixture a product in which sodium hyaluronate, hydroxymethylcellulose or fragrance, are added to the sulfated cellulose compounds and fine sorbitol powder, adding sodium sulfate anhydrous thereto, and stirring the mixture homogeneously. However, the production method is not limited thereto.

In the case of patch, it may be prepared, for example, by dissolving 1-menthol, polyacrylic acid or carboxymethylcellulose sodium, in glycerin, further adding sorbitol or caster oil, thereto, adding to the mixture purified water in which the sulfated cellulose compounds are dissolved, and stirring the mixture homogeneously. However, the production method is not limited thereto.

EXAMPLES

Hereinafter, the present invention will be described in more detail based on working examples, but the present invention is not limited thereto.

[Synthesis Example 1]

A 2000-mL three-necked flask equipped with a mechanical stirrer and a thermometer was charged with 50.5 g of Ceolus which was dried at 60°C for 7 hours (crystalline cellulose having the polymerization degree of 100 to 300; manufactured by Asahi Kasei Chemicals Corporation) and 166 g of N,N-dimethylformamide, and the mixture was stirred at room temperature (25°C) for 1 hour. Next, 482 g of N,N-dimethylformamide solution having 19.7 wt% of complex of N,N-dimethylformamide and sulfuric anhydride (the complex: $620 \times 10^{-3}$ mol) was added dropwise to the mixture for 9 minutes, retaining the temperature of the content in the flask so that it did not exceed 25°C. After retaining at room temperature for 24 hours, the flask was iced, and 400 g of water was added dropwise to the reaction solution. Further, using 5M sodium hydroxide aqueous solution (165 g), pH of the mixture was adjusted to 10, and after that, using 1M HCl aqueous solution (4.3 g), pH of the reaction solution was adjusted to 7. Next, salts and impurities precipitated were removed by filtration. The obtained filtrate was added dropwise to 2500 g of 2-propanol over 50 minutes, and after that, the mixture was stirred for 15 minutes. Crystal precipitated was obtained by filtration, and washing operation using 400 g of methanol was carried out on a funnel four times. The obtained crystal was suspended in 400 g of methanol, and thereafter allowed to stand overnight. After the crystal was obtained by filtration, it was dried under reduced pressure at room temperature to obtain 96.2 g of product of interest, sodium cellulose sulfate. As a result of elemental analysis, the sulfur concentration was 15.2 wt% and the sodium concentration was 10.6 wt%. The weight average molecular weight of the compound was 75000, and the solubility was 10 g/L or higher.

The N,N-dimethylformamide solution having 19.7 wt% of complex of N,N-dimethylformamide and sulfuric anhydride was prepared as described below

A 5-L round-bottomed flask was charged with 2000 g of N,N-dimethylformamide, and it was cooled to -10°C or lower. 500 g of sulfuric anhydride was added dropwise thereto under stirring. This was performed over about 2 hours with the temperature being adjusted to 0°C or lower. The mixture was gradually warmed to room temperature, and in order to dissolve precipitate, some amount of N,N-dimethylformamide was added to the mixture. As a result, a light yellow transparent solution was obtained (yield: 2538 g).

[Example 1]

Sodium cellulose sulfate obtained in Synthesis Example 1 was diluted with 0.1% aqueous solution of glycerin to be prepared to have the concentration of 0.015 mg/mL.

[Comparative Example 1]

Sodium cellulose sulfate obtained in Synthesis Example 1 was diluted with ion exchange water to be prepared to have the concentration of 0.015 mg/mL.

<Examination regarding inhibition of activity of hyaluronidase derived from bovine testis>

Each of the prepared products in Example 1 and Comparative Example 1 was subjected to examination regarding inhibition of activity of hyaluronidase derived from bovine testis. The reagent used in the examination is manufactured by Wako Pure Chemical Industries, Ltd., unless otherwise specified.
The following 6 types of solutions were prepared:

Solution A: 0.1 mol/L acetic acid buffer (pH 4.0) solution (concentration: 2.83 mg/mL) of hyaluronidase (manufactured by Sigma) originating in a bovine testicle

Solution B: 0.3 mol/L sodium chloride - 0.1 mol/L acetic acid buffer solution (pH 4.0)

Solution C: 0.1 mol/L acetic acid buffer (pH 4.0) solution (concentration: 1.83 mg/mL) of sodium hyaluronate (manufactured by Chisso corporation)

Solution D: 0.4 mol/L sodium hydroxide aqueous solution

Solution E: 0.8 mol/L sodium borate aqueous solution

Solution F: solution prepared by adding 1.25 mL of 10N hydrochloric acid and 98.75 mL of acetic acid to 1 g of para-dimethylaminobenzaldehyde

(Preparation of test solutions)

0.025 mL of Solution A was mixed with 0.2 mL of Solution B, and the mixture was retained at 37°C for 20 minutes. To this mixture, each of the solutions with different concentrations prepared in Example 1 and Comparative Example 1 was added independently, and allowed to stand in a constant-temperature bath at 37°C for 20 minutes. 0.2 mL of Solution C was further added to each of the mixtures, and allowed to stand in a constant-temperature bath at 37°C for 20 minutes. Subsequently, 0.1 mL of Solution D and 0.1 mL of Solution E were added to each of the mixtures, and boiled for 3 minutes and thereafter cooled. After that, 3.0 mL of Solution F was added to each of the mixtures, and allowed to stand in a constant-temperature bath at 37°C for 20 minutes, thereby preparing test solutions. The absorbance $Q_E$ of the test solutions at 585 nm was measured using N-acetylhexosamine of a reduction end produced by degradation of hyaluronidase as an index, and pure water was used as a control.

(Preparation of Control solution 1)

Control solution 1 was prepared in a manner similar to that for the preparation of the above-described test solutions except that 0.1 mol/L acetate buffer solution (pH 4.0) was used instead of Solution A and pure water was used instead of aqueous solution of sodium cellulose sulfate. The absorbance $Q_1$ of Control solution 1 at 585 nm was measured as in the case of the test solutions.

(Preparation of Control solution 2)

Control solution 2 was prepared in a manner similar to that for the preparation of the above-described test solutions except that pure water was used instead of aqueous solution of sodium cellulose sulfate. The absorbance $Q_2$ of Control solution 2 at 585 nm was measured as in the case of the test solutions.
The hyaluronidase activity inhibition ratio was calculated according to the formula described below. The hyaluronic acid degradation activity of each of a system including sodium cellulose sulfate and polyhydric alcohol, prepared in Example 1, and a system including only sodium cellulose sulfate prepared in Comparative Example 1 was examined.

$$\text{Inhibition ratio (\%)} = \{(Q_2 - Q_1) - (Q_E - Q_1)\}/(Q_2 - Q_1)$$

Results are shown in Table 1.

Table 1

| | Example 1 | Comparative Example 1 |
|---|---|---|
| Inhibition ratio (%) in the case of 0.015mg/mL | 68.1 | 64.5 |

According to the results in Table 1, it is understood that the system including sodium cellulose sulfate and polyhydric alcohol has higher hyaluronidase inhibition activity compared to the system including only sodium cellulose sulfate. The hyaluronidase inhibition activity of Example 1 was 1.05 times higher than that of Comparative Example 1. Therefore, it became clear that, by using sodium cellulose sulfate and polyhydric alcohol in combination, a cosmetic composition exerts an effect of reducing degradation of hyaluronic acid due to hyaluronidase to improve moisture-retaining property.

[Example 2]

Using 0.50% aqueous solution of glycerin instead of 0.1% aqueous solution of glycerin, a solution of sodium cellulose sulfate having the concentration of 0.015 mg/mL was prepared according to Example 1. The prepared solution was subjected to examination of inhibition activity for hyaluronidase derived from bovine testis. As a result, the activity was 1.02 times higher than that of Comparative Example 1.

[Example 3]

Using 10.0% aqueous solution of glycerin instead of 0.1% aqueous solution of glycerin, a solution of sodium cellulose sulfate having the concentration of 0.015 mg/mL was prepared according to Example 1. The prepared solution was subjected to examination of inhibition activity for hyaluronidase derived from bovine testis. As a result, the activity was 1.07 times higher than that of Comparative Example 1.

[Example 4]

Using aqueous solution of 1,2-propanediol instead of aqueous solution of glycerin, solutions of sodium cellulose sulfate having the concentration of 0.015 mg/mL were prepared according to Example 1. As three concentrations of aqueous solution of 1,2-propanediol, 0.10%, 0.50% and 10.0% were employed. Then prepared solutions were independently subjected to examination of inhibition activity for hyaluronidase derived from bovine testis. As a result, the relative activity with respect to Comparative Example 1 was 1.02 times, 1.02 times and 1.03 times, respectively.

[Example 5]

Using aqueous solution of 1,3-butanediol instead of aqueous solution of glycerin, solutions of sodium cellulose sulfate having the concentration of 0.015 mg/mL were prepared according to Example 1. As three concentrations of aqueous solution of 1,3-butanediol, 0.10%, 0.50% and 10.0% were employed. The prepared solutions were independently subjected to examination of inhibition activity for hyaluronidase derived from bovine testis. As a result, the relative activity with respect to Comparative Example 1 was 1.02 times, 1.03 times and 1.03 times, respectively.

Results are shown in Table 2.

Table 2

| Concentration of aqueous solution of polyhydric alcohol | 0.0% | 0.10% | 0.50% | 10.0% |
|---|---|---|---|---|
| glycerin | 1.00 | 1.05 | 1.02 | 1.07 |
| 1,2-propanediol | 1.00 | 1.02 | 1.02 | 1.03 |
| 1,3-butanediol | 1.00 | 1.02 | 1.03 | 1.03 |

(Production Examples of Cosmetic Compositions)

Various types of cosmetic compositions were produced according to the present invention. Some examples are given below, but the present invention is not limited to these examples.

| (Production Example 1) Milky lotion | wt% |
|---|---|
| polyether-modified organopolysiloxane | 5.0 |
| diglyceryl monostearate | 1.5 |
| decamethylcyclotetrasiloxane | 20.0 |
| dimethylpolysiloxane | 10.0 |
| liquid paraffin | 10.0 |
| fragrance | 0.1 |
| compound obtained in Synthesis Example 1 | 0.1 |
| 1,3-butanediol | 15.0 |
| sodium chloride | 0.2 |
| purified water | balance |

| (Production Example 2) Serum | wt% |
|---|---|
| sorbitan sesquiisostearate | 3.0 |
| decamethylcyclopentasiloxane | 10.0 |
| isononyl isomyristate | 8.0 |
| perfluoropolyether | 0.5 |
| fragrance | 0.1 |
| compound obtained in Synthesis Example 1 | 0.05 |
| glycerin | 3.0 |
| 1,2-propanediol | 10.0 |
| ascorbic acid 2-glucoside | 3.0 |
| sodium citrate | 0.5 |
| purified water | balance |

| (Production Example 3) Cream | wt% |
|---|---|
| petrolatum | 8.0 |
| N-(3-hexadecyloxy-2-hydroxypropyl) -N-2-hydroxyethylhexadecanamide | 2.0 |
| squalene | 20.0 |
| cetyl alcohol | 5.0 |
| glycerin monostearate | 2.0 |
| polyoxyethylene (20) sorbitan monolaurate | 2.0 |
| arginine | 0.1 |
| ceramide 3 | 0.5 |
| sodium hydrogen phosphate | 0.85 |
| compound obtained in Synthesis Example 1 | 0.2 |
| glycerin | 5.0 |
| 1,3-butanediol | 5.0 |
| dimethylpolysiloxane | 3.0 |
| fragrance | 0.1 |
| purified water | balance |

| (Production Example 4) Toner | wt% |
|---|---|
| 1,3-butanediol | 5.0 |
| methyl parahydroxybenzoate | 0.13 |

(continued)

| (Production Example 4) Toner | wt% |
|---|---|
| propyl parahydroxybenzoate | 0.02 |
| glycerin | 3.0 |
| compound obtained in Synthesis Example 1 | 0.1 |
| citric acid | 0.009 |
| sodium citrate | 0.13 |
| purified water | balance |

| (Production Example 5) Toner | wt% |
|---|---|
| 1,3-butanediol | 5.0 |
| methyl parahydroxybenzoate | 0.13 |
| propyl parahydroxybenzoate | 0.02 |
| sodium hyaluronate | 0.02 |
| glycerin | 3.0 |
| compound obtained in Synthesis Example 1 | 0.1 |
| citric acid | 0.008 |
| sodium citrate | 0.1 |
| purified water | balance |

| (Production Example 6) Toner | wt% |
|---|---|
| 1,3-butanediol | 5.0 |
| methyl parahydroxybenzoate | 0.13 |
| propyl parahydroxybenzoate | 0.02 |
| sodium hyaluronate | 0.02 |
| glycerin | 3.0 |
| compound obtained in Synthesis Example 1 | 0.5 |
| sodium citrate | 0.3 |
| purified water | balance |

| (Production Example 7) Foundation | wt% |
|---|---|
| decamethylcyclopentasiloxane | 45.0 |
| dimethylpolysiloxane | 5.0 |
| dimethyldistearyl ammonium hectorite | 4.0 |
| hydrophobic treated titanium oxide | 10.0 |
| hydrophobic treated talc | 6.0 |
| hydrophobic treated mica | 6.0 |
| hydrophobic treated red iron oxide | 1.6 |
| hydrophobic treated yellow iron oxide | 0.7 |
| hydrophobic treated black iron oxide | 0.2 |
| compound obtained in Synthesis Example 1 | 0.1 |
| dipropylene glycol | 5.0 |
| 2-amino-2-methyl-1,3-propanediol | 0.5 |
| fragrance | 0.1 |
| purified water | balance |

| (Production Example 8) Liquid foundation | wt% |
|---|---|
| decamethylcyclopentasiloxane | 16.0 |
| dimethylpolysiloxane | 8.0 |
| 12-hydroxystearic acid | 1.0 |
| fluorine-modified silicone | 5.0 |
| spherical silicone resin powder | 3.0 |
| fluorine compound-treated titanium oxide fine powder | 8.0 |
| fluorine compound-treated mica-titanium | 1.0 |
| fluorine compound-treated titanium oxide | 5.0 |
| fluorine compound-treated red iron oxide | 0.9 |
| fluorine compound-treated yellow iron oxide | 2.0 |
| fluorine compound-treated black iron oxide | 1.0 |
| ethanol | 15.0 |
| compound obtained in Synthesis Example 1 | 0.5 |
| 1,6-hexanediol | 2.0 |
| glycerin | 3.0 |
| magnesium sulfate | 1.0 |
| fragrance | 0.05 |
| purified water | balance |

| (Production Example 9) Lipstick | wt% |
|---|---|
| cadelin wax | 5.0 |
| ceresin wax | 15.0 |
| beeswax | 3.0 |
| decamethylcyclopentasiloxane | 15.0 |
| propylene glycol dicaprate | 10.0 |
| polyglyceryl diisostearate | 8.0 |
| sorbitan sesquioleate | 1.0 |
| polyether-modified organopolysiloxane | 2.0 |
| compound obtained in Synthesis Example 1 | 0.05 |
| 1,3-butanediol | 1.0 |
| Red No. 201 | 1.5 |
| Yellow No. 401 | 1.0 |
| hydrophobic treated titanium oxide | 1.0 |
| hydrophobic treated mica-titanium | 5.0 |
| fragrance | 1.0 |
| cetyl isooctanoate | balance |

| (Production Example 10) Eye shadow | wt% |
|---|---|
| decamethylsiloxane | 15.0 |
| dimethylpolysiloxane | 10.0 |
| polyethylene glycol (10) lauryl ether | 0.5 |
| silicone-treated chromium oxide | 6.5 |
| silicone-treated ultramarine | 4.0 |
| silicone-treated titanium-coated mica | 6.0 |
| sodium chloride | 2.0 |
| propylene glycol | 8.0 |
| compound obtained in Synthesis Example 2 | 0.5 |

(continued)

| (Production Example 10) Eye shadow | wt% |
|---|---|
| fragrance | 0.1 |
| purified water | balance |

| (Production Example 11) Facial wash | wt% |
|---|---|
| triethanolamine lauryl phosphate | 18.0 |
| triethanolamine laurate | 2.5 |
| triethanolamine myristate | 2.5 |
| compound obtained in Synthesis Example 1 | 0.2 |
| glycerin | 16.0 |
| polyoxyethylene (160) sorbitan triisostearate | 2.0 |
| cationated cellulose | 0.5 |
| alkyl acrylate/methacrylate copolymer | 0.5 |
| lauryl hydroxysulfobetaine | 5.0 |
| fragrance | 0.1 |
| purified water | balance |

| (Production Example 12) Body shampoo | wt% |
|---|---|
| sodium citrate | 2.0 |
| N-acetylglutamic acid | 1.0 |
| potassium laurate | 15.0 |
| potassium myristate | 5.0 |
| compound obtained in Synthesis Example 1 | 0.3 |
| 1,2-propanediol | 5.0 |
| sorbitol | 3.0 |
| methylpolysiloxane | 1.5 |
| amino-modified dimethylpolysiloxane | 2.5 |
| polyethylene powder | 0.5 |
| hydroxypropyl chitosan | 0.5 |
| fragrance | 0.1 |
| purified water | balance |

| (Production Example 13) hair dressing | wt% |
|---|---|
| vinylpyrrolidone/N,N-dimethylaminoethylmethacrylic acid copolymer diethyl sulfate | 1.0 |
| polyoxyethylene hydrogenated caster oil | 0.3 |
| keratin hydrolysate | 1.0 |
| hydroxypropyl chitosan | 3.5 |
| acetylneuraminic acid | 0.1 |
| compound obtained in Synthesis Example 1 | 0.05 |
| mannitol | 4.0 |
| 1,3-butanediol | 5.0 |
| ethanol | 10.0 |
| fragrance | 0.1 |
| purified water | balance |

| (Production Example 14) Shampoo | wt% |
|---|---|
| stearyl dimethylamine | 0.1 |
| cetyl dimethylamine | 0.1 |
| cetyl alcohol | 0.5 |
| carboxyvinyl polymer | 0.4 |
| isopropyl palmitate | 1.6 |
| dimethylpolysiloxane | 0.7 |
| dimethylpolysiloxane | 0.3 |
| lauryldimethylamine oxide | 10.0 |
| lauryl carboxymethyl hydroxyethyl imidazolinium betaine | 1.0 |
| cationated cellulose | 0.5 |
| compound obtained in Synthesis Example 1 | 0.5 |
| 1,2-propanediol | 0.5 |
| ethylene glycol distearate | 2.0 |
| sodium citrate | 0.2 |
| fragrance | 0.1 |
| purified water | balance |

| (Production Example 15) Finishing rinse | wt% |
|---|---|
| dialkyl dimethyl ammonium chloride | 3.0 |
| polyoxyethylene (5) lauryl ether | 5.0 |
| isostearyl glyceryl ether | 2.0 |
| compound obtained in Synthesis Example 1 | 0.8 |
| 1,2-pentanediol | 1.5 |
| hydroxypropyl cellulose | 0.4 |
| isopropyl palmitate | 1.6 |
| dimethylpolysiloxane | 0.1 |
| citric acid | 0.5 |
| fragrance | 0.1 |
| purified water | balance |

| (Production Example 16) Hair treatment | wt% |
|---|---|
| behenyl trimethyl ammonium chloride | 8.0 |
| behenyl alcohol | 7.0 |
| carboxymethyl chitin | 1.0 |
| isopropyl palmitate | 9.0 |
| dimethylpolysiloxane | 1.0 |
| polyoxyethylene (20) sorbitan monostearate | 0.5 |
| behenic acid | 1.0 |
| compound obtained in Synthesis Example 1 | 1.0 |
| dipropylene glycol | 6.0 |
| glycerin | 10.0 |
| citric acid | 0.3 |
| fragrance | 0.1 |
| purified water | balance |

| (Production Example 17) Hair dye | wt% |
|---|---|
| (First Solution) sodium citrate | 0.8 |
| aqueous ammonia solution (30%) | 5.0 |
| 2,5-diaminotoluene sulfate salt | 5.0 |
| 2-amino-4-nitrophenol | 3.0 |
| 5-aminoorthocresol | 1.0 |
| p-aminophenol | 1.0 |
| 2,6-diaminopyridine | 0.1 |
| hydroxyethyl cellulose | 2.5 |
| oleyl alcohol | 5.0 |
| compound obtained in Synthesis Example 1 | 0.05 |
| 1,2-propanediol | 2.0 |
| pantothenyl ethyl ether | 0.2 |
| sodium sulfite | 1.0 |
| tetrasodium edentate | 0.2 |
| monoethanol amine | 1.5 |
| purified water | balance |

| (Second Solution) | |
|---|---|
| aqueous hydrogen peroxide(35%) | 15.0 |
| compound obtained in Synthesis Example 1 | 0.05 |
| 1,3-butanediol | 10.0 |
| stearyl trimethyl ammonium chloride | 0.5 |
| lactic acid | 0.2 |
| fragrance | 0.1 |
| purified water | balance |

| (Production Example 18) Hair growth tonic | wt% |
|---|---|
| polyoxypropylene diglyceryl ether | 3.0 |
| polyoxyethylene polyoxypropylene glycol | 2.0 |
| glutamic acid sodium | 2.0 |
| ethanol | 60.0 |
| compound obtained in Synthesis Example 1 | 0.05 |
| sorbitol | 5.0 |
| 1,5-pentanediol | 4.5 |
| DL-alpha-tocopherol acetate | 1.5 |
| capsicum tincture | 0.5 |
| resorcin | 0.5 |
| dipotassium glycyrrhizinate | 0.5 |
| carboxymethyl chitin | 0.5 |
| decamethylcyclopentasiloxane | 0.1 |
| purified water | balance |

| (Production Example 19) Bath agents | wt% |
|---|---|
| sodium hydrogen carbonate | 60.0 |
| sodium borate | 5.0 |
| sodium hyaluronate | 1.0 |
| hydroxypropylmethyl cellulose | 1.0 |

(continued)

| (Production Example 19) Bath agents | wt% |
|---|---|
| fine sorbitol powder | 5.0 |
| compound obtained in Synthesis Example 1 | 0.5 |
| fragrance | 3.0 |
| sodium sulfate anhydrous | balance relative to 100 |

| (Production Example 20) Ointment | wt% |
|---|---|
| 1-menthol | 3.0 |
| DL-camphor | 0.5 |
| compound obtained in Synthesis Example 1 | 0.2 |
| 1,2-propanediol | 15.0 |
| carboxyvinyl polymer | 1.0 |
| hydroxyethyl cellulose | 0.1 |
| triethanol amine | 0.7 |
| purified water | 25.0 |
| ethanol | balance relative to 100 |

| (Production Example 21) Patch | wt% |
|---|---|
| 1-menthol | 3.0 |
| DL-camphor | 1.5 |
| polyacrylic acid | 4.5 |
| sodium polyacrylate | 1.5 |
| carboxymethylcellulose sodium | 4.0 |
| compound obtained in Synthesis Example 1 | 0.1 |
| glycerin | 20.0 |
| sorbitol | 5.0 |
| polyoxyethylene nonylphenyl ether | 0.5 |
| kaolin | 5.0 |
| caster oil | 1.0 |
| purified water | balance |

The aforementioned components were dissolved, dispersed, and compounded, which was spread over a polyester non-woven fabric at 1,000 g per 1 m$^2$ to produce a patch.

| (Production Example 22) Toner | wt% |
|---|---|
| 1,3-butanediol | 5.0 |
| glycerin | 3.0 |
| methylparaben | 0.10 |
| propylparaben | 0.01 |
| compound obtained in Synthesis Example 1 | 0.5 |
| citric acid | 0.01 |
| sodium citrate | 0.10 |
| purified water | balance |

[Stability obtained from pH adjustment]

Next, stability of the cosmetic composition obtained from pH adjustment was evaluated using the toner of Production Example 22 in which pH was adjusted by addition of citric acid and sodium citrate.

1.5 g of sodium cellulose sulfate obtained in Synthesis Example 1 was dissolved in purified water (300 mL in total) to prepare 0.5% aqueous solution of sodium cellulose sulfate as a control solution.

Each of the toner of Production Example 22 and 0.5% aqueous solution of sodium cellulose sulfate was divided into 9 equal parts. 9 equal parts were divided into 3 groups consisting of 3 equal parts, and this 3 groups were allowed to stand in 3 types of constant-temperature containers whose temperatures were set at 20°C, 40°C and 60°C, respectively. 1, 2, 9, 16 and 23 days later, pH of each solution was measured, and the average values were calculated. Results are shown in Table 3.

Table 3

|  | Preset temperature | First day | 1 day later | 2 days later | 9 days later | 16 days later | 23 days later |
|---|---|---|---|---|---|---|---|
| 0.5% aqueous solution of compound obtained in Synthesis Example 1 | 20°C | 6.36 | 6.25 | 6.35 | 6.34 | 6.17 | 5.98 |
|  | 40°C | 6.36 | 6.74 | 6.49 | 6.19 | 6.01 | 5.67 |
|  | 60°C | 6.36 | 6.11 | 5.69 | 2.22 | 2.10 | 2.06 |
| (Production Example 22) Toner | 20°C | 6.18 | 6.17 | 6.17 | 6.17 | 6.25 | 6.24 |
|  | 40°C | 6.18 | 6.16 | 6.14 | 6.07 | 6.24 | 6.21 |
|  | 60°C | 6.18 | 6.12 | 6.09 | 6.06 | 6.09 | 6.04 |

The toner of Production Example 22 in which pH was adjusted using citric acid and sodium citrate showed better stability compared to the aqueous solution in which pH was not adjusted.

INDUSTRIAL APPLICABILITY

The cosmetic composition of the present invention has a moisturizing effect and high ability to inhibit hyaluronidase activity, and can reduce skin roughness caused by drying. Therefore, the composition may be utilized in various product forms. The cosmetic composition of the present invention can be provided to people with sensitive skin, who are highly sensitive to chemical substances contained in cosmetics.

**Claims**

1. A cosmetic composition comprising: one or more substances selected from sulfated cellulose and a salt thereof; and polyhydric alcohol, wherein sulfated cellulose and the salt thereof have: the weight average molecular weight of 1,000 to 200,000; the sulfur concentration of 6.5 to 19.0 wt%; and the solubility in pure water at 20°C of 3g/L or higher.

2. The cosmetic composition according to claim 1, wherein the salt of sulfated cellulose is one or a combination of two or more of substances selected from lithium salt, potassium salt, sodium salt, beryllium salt, magnesium salt, calcium salt, triethylamine salt, arginine salt, lysine salt and histidine salt.

3. The cosmetic composition according to claim 1 or 2, comprising 0.001 to 20 wt% of one or more substances selected from sulfated cellulose and the salt thereof and 0.0001 to 80 wt% of polyhydric alcohol.

4. The cosmetic composition according to any one of claims 1 to 3, wherein sulfated cellulose is obtained by sulfating crystalline cellulose having the average degree of polymerization of 100 to 300 using sulfuric anhydride or a complex of N,N-dimethylformamide and sulfuric anhydride.

5. The cosmetic composition according to any one of claims 1 to 4, wherein polyhydric alcohol is one or a combination of two or more of substances selected from the group consisting of 1,2-ethanediol, diethylene glycol, triethylene glycol, polyethylene glycol, 1,2-propanediol, 1,3-propanediol, dipropylene glycol, polypropylene glycol, 1,2-butanediol, 1,3-butanediol, 1,4-butanediol, 3-methyl-1,3-butanediol, glycerin, 1,2-pentanediol, 1,3-pentanediol, 1,4-pentanediol, 1,5-pentanediol, 1,2,3-pentanetriol, 2,3,4-pentanetriol, 1,3,4-pentanetriol, 1,3,5-pentanetriol, 2-methyl-2,4-pentanediol, 1,2-hexanediol, 1,3-hexanediol, 1,4-hexanediol, 1,5-hexanediol, 1,6-hexanediol, 1,2,3-hexanetriol, 1,3,4-hexanetriol, 1,3,5-hexanetriol, 1,4,6-hexanetriol, erythritol, pentaerythritol, dipentaerythritol, mannitol, sorbitol, threitol, arabitol, xylitol, ribitol, galactitol, lactitol, maltitol, inositol, panthenol, cyclitol, laminitol, valienamine, validamine, validanol and 2-amino-2-methyl-1,3-propanediol.

6. The cosmetic composition according to any one of claims 1 to 5, further comprising an electrolyte having pH buffering ability.

7. The cosmetic composition according to claim 6, wherein the content of the electrolyte having pH buffering ability in the cosmetic composition is 0.001 to 30 wt%.

8. The cosmetic composition according to any one of claims 1 to 7, wherein pH of the cosmetic composition is 5.5 to 7.0.

9. The cosmetic composition according to any one of claims 1 to 8, wherein the cosmetic composition is a hair treating agent.

10. The cosmetic composition according to any one of claims 1 to 8, wherein the cosmetic composition is a skin care cosmetic.

11. The cosmetic composition according to any one of claims 1 to 8, wherein the cosmetic composition is a makeup cosmetic.

12. The cosmetic composition according to any one of claims 1 to 8, wherein the cosmetic composition is a skin cleanser.

13. The cosmetic composition according to any one of claims 1 to 8, wherein the cosmetic composition is a bath agent.

14. The cosmetic composition according to any one of claims 1 to 8, wherein the cosmetic composition is a patch.

**Patentansprüche**

1. Kosmetische Zusammensetzung aufweisend: eine oder mehrere Substanzen ausgewählt aus sulfatierter Cellulose und einem Salz davon; und mehrwertigem Alkohol, wobei die sulfatierte Cellulose und das Salz davon aufweisen: das gewichtsmittlere Molekulargewicht von 1.000 bis 200.000; die Schwefelkonzentration von 6,5 bis 19,0 Gew%; und die Löslichkeit in reinem Wasser bei 20 ° C von 3 g/l oder höher.

2. Kosmetische Zusammensetzung nach Anspruch 1, wobei das Salz der sulfatierten Cellulose eine oder eine Kombination von zwei oder mehreren Substanzen ausgewählt aus Lithiumsalz, Kaliumsalz, Natriumsalz, Berylliumsalz, Magnesiumsalz, Calciumsalz, Triethylaminsalz, Argininsalz, Lysinsalz und Histidinsalz ist.

3. Kosmetische Zusammensetzung nach Anspruch 1 oder 2 aufweisend 0,001 bis 20 Gew-% einer oder mehrerer Substanzen ausgewählt aus sulfatierter Cellulose und dem Salz davon und 0,0001 bis 80 Gew-% mehrwertigem Alkohol.

4. Kosmetische Zusammensetzung nach einem der Ansprüche 1 bis 3, wobei die sulfatierte Cellulose durch Sulfatisieren von kristalliner Cellulose mit dem mittleren Polymerisationsgrad von 100 bis 300 unter Verwendung von Schwefelsäureanhydrid oder einem Komplex von N, N-Dimethylformamid und Schwefelsäureanhydrid erhalten wird.

5. Kosmetische Zusammensetzung nach einem der Ansprüche 1 bis 4, wobei der mehrwertige Alkohol eine oder eine Kombination von zwei oder mehreren Substanzen ist, ausgewählt aus der Gruppe bestehend aus 1,2-Ethandiol, Diethylenglykol, Triethylenglykol, Polyethylenglykol, 1,2-Propandiol, 1,3-Propandiol, Dipropylenglykol, Polypropylenglykol, 1,2-Butandiol, 1,3-Butandiol, 1,4-Butandiol, 3-Methyl-1,3-Butandiol, Glycerin, 1,2-Pentandiol, 1,3-Pentandiol, 1,4-Pentandiol, 1,5-Pentandiol, 1,2,3-Pentantriol, 2,3,4-Pentantriol, 1,3,4-Pentantriol, 1,3,5-Pentantriol, 2-Methyl-2,4-Pentandiol, 1,2-Hexandiol, 1,3-Hexandiol, 1,4-Hexandiol, 1,5-Hexandiol, 1,6-Hexandiol, 1,2,3-Hexantriol, 1,3,4-Hexantriol, 1,3,5-Hexantriol, 1,4,6-Hexantriol, Erythrit, Pentaerythrit, Dipentaerythrit, Mannit, Sorbit, Threit, Arabit, Xylit, Ribit, Galactit, Lactit, Maltit, Inosit, Panthenol, Cycilitol, Laminitol, Valienamin, Validamin, Validanol und 2-Amino-2-Methyl-1,3-Propandiol.

6. Kosmetische Zusammensetzung nach einem der Ansprüche 1 bis 5, ferner aufweisend einen Elektrolyt mit pH-Pufferfähigkeit.

7. Kosmetische Zusammensetzung nach Anspruch 6, wobei der Gehalt des Elektrolyten mit pH-Pufferfähigkeit in der kosmetischen Zusammensetzung 0,001 bis 30 Gew-% beträgt.

8. Kosmetische Zusammensetzung nach einem der Ansprüche 1 bis 7, wobei der pH der kosmetischen Zusammensetzung 5,5 bis 7,0 beträgt.

9. Kosmetische Zusammensetzung nach einem der Ansprüche 1 bis 8, wobei die kosmetische Zusammensetzung ein Haarbehandlungsmittel ist.

10. Kosmetische Zusammensetzung nach einem der Ansprüche 1 bis 8, wobei die kosmetische Zusammensetzung eine kosmetische Hautpflege ist.

11. Kosmetische Zusammensetzung nach einem der Ansprüche 1 bis 8, wobei die kosmetische Zusammensetzung ein Make-up-Kosmetikum ist.

12. Kosmetische Zusammensetzung nach einem der Ansprüche 1 bis 8, wobei die kosmetische Zusammensetzung ein Hautreinigungsmittel ist.

13. Kosmetische Zusammensetzung nach einem der Ansprüche 1 bis 8, wobei die kosmetische Zusammensetzung eine Pflegebad ist.

14. Kosmetische Zusammensetzung nach einem der Ansprüche 1 bis 8, wobei die kosmetische Zusammensetzung ein Pflaster ist.


**Revendications**

1. Composition cosmétique comprenant : une ou plusieurs substances choisies parmi la cellulose sulfatée et un sel de celle-ci ; et un alcool polyhydrique, la cellulose sulfatée et le sel de celle-ci ayant : un poids moléculaire moyen en poids de 1 000 à 200 000 ; une concentration en soufre de 6,5 à 19,0 % en poids ; et une solubilité dans l'eau pure à 20 °C de 3 g/l ou plus.

2. Composition cosmétique selon la revendication 1, dans laquelle le sel de cellulose sulfatée est une substance ou une combinaison de deux substances ou plus choisies parmi un sel de lithium, un sel de potassium, un sel de sodium, un sel de béryllium, un sel de magnésium, un sel de calcium, un sel de triéthylamine, un sel d'arginine, un sel de lysine et un sel d'histidine.

3. Composition cosmétique selon la revendication 1 ou 2, comprenant 0,001 à 20 % en poids d'une ou de plusieurs substances choisies parmi la cellulose sulfatée et le sel de celle-ci et 0,0001 à 80 % en poids d'alcool polyhydrique.

4. Composition cosmétique selon l'une quelconque des revendications 1 à 3, dans laquelle la cellulose sulfatée est obtenue par sulfatation de cellulose cristalline ayant un degré moyen de polymérisation de 100 à 300 en utilisant de l'anhydride sulfurique ou un complexe de N,N-diméthylformamide et d'anhydride sulfurique.

5. Composition cosmétique selon l'une quelconque des revendications 1 à 4, dans laquelle l'alcool polyhydrique est une substance ou une combinaison de deux substances ou plus choisies dans le groupe constitué par le 1,2-éthanediol, le diéthylène glycol, le triéthylène glycol, le polyéthylène glycol, 1,2-propanediol, le 1,3-propanediol, le dipropylène glycol, le polypropylène glycol, le 1,2-butanediol, le 1,3-butanediol, le 1,4-butanediol, le 3-méthyl-1,3-butanediol, la glycérine, le 1,2-pentanediol, le 1,3-pentanediol, le 1,4-pentanediol, le 1,5-pentanediol, le 1,2,3-pentanetriol, le 2,3,4-pentanetriol, le 1,3,4-pentanetriol, le 1,3,5-pentanetriol, le 2-méthyl-2,4-pentanediol, le 1,2-hexanediol, le 1,3-hexanediol, le 1,4-hexanediol, le 1,5-hexanediol, le 1,6-hexanediol, le 1,2,3-hexanetriol, le 1,3,4-hexanetriol, le 1,3,5-hexanetriol, le 1,4,6-hexanetriol, l'érythritol, le pentaérythritol, le dipentaérythritol, le mannitol, le sorbitol, le thréitol, l'arabitol, le xylitol, le ribitol, le galactitol, le lactitol, le maltitol, l'inositol, le panthénol, le cyclitol, le laminitol, la valiénamine, la validamine, le validanol et le 2-amino-2-méthyl-1,3-propanediol.

6. Composition cosmétique selon l'une quelconque des revendications 1 à 5, comprenant en outre un électrolyte ayant une capacité de tamponnage du pH.

7. Composition cosmétique selon la revendication 6, dans laquelle la teneur de l'électrolyte ayant une capacité de tamponnage du pH dans la composition cosmétique est de 0,001 à 30 % en poids.

8. Composition cosmétique selon l'une quelconque des revendications 1 à 7, dans laquelle le pH de la composition cosmétique est de 5,5 à 7,0.

9. Composition cosmétique selon l'une quelconque des revendications 1 à 8, dans laquelle la composition cosmétique est un agent de traitement capillaire.

10. Composition cosmétique selon l'une quelconque des revendications 1 à 8, dans laquelle la composition cosmétique est un cosmétique de soin de la peau.

11. Composition cosmétique selon l'une quelconque des revendications 1 à 8, dans laquelle la composition cosmétique est un cosmétique de maquillage.

12. Composition cosmétique selon l'une quelconque des revendications 1 à 8, dans laquelle la composition cosmétique est un produit de nettoyage pour la peau.

13. Composition cosmétique selon l'une quelconque des revendications 1 à 8, dans laquelle la composition cosmétique est un agent pour le bain.

14. Composition cosmétique selon l'une quelconque des revendications 1 à 8, dans laquelle la composition cosmétique est un patch.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006274245 A
- WO 0048568 A1
- WO 698641 A1
- US 5609723 A1
- JP 2000178196 A
- JP 2005350454 A
- EP 1604647 A